# EUROPEAN PATENT APPLICATION

(11) **EP 4 272 797 A1**
(43) Date of publication of application: **08.11.2023**
(21) Application number: 22748817.8
(22) Date of filing: 11.01.2022
(51) Int. Cl.: A61M 25/00

(54) **CATHETER REINFORCEMENT LAYER AND CATHETER**

(30) Priority: 05.02.2021 CN 202110164303
(71) Applicant: MicroPort NeuroTech (Shanghai) Co., Ltd., Shanghai 201318 (CN)
(72) Inventor: LIN, Heng, Shanghai 201318 (CN); LIU, Yunyun, Shanghai 201318 (CN); CUN, Yuxi, Shanghai 201318 (CN); SUN, Li, Shanghai 201318 (CN)
(74) Representative: Marks & Clerk LLP
(86) International application number: PCT/CN2022/071230
(87) International publication number: WO 2022/166538

(57) **Abstract**

The present invention provides a catheter reinforcement layer (100) and a catheter including the same. The catheter reinforcement layer (100) includes a braided component (110) and at least one axial component (120a, 120b, 120c, 120d, 120e, 120f, 120g, 120h, 120j, 120k, 1201, 120m, 120n). The braided component (110) is a mesh tube formed by braiding wires in a crosswise manner, and the axial component (120a, 120b, 120c, 120d, 120e, 120f, 120g, 120h, 120j, 120k, 1201, 120m, 120n) extends along the braided component (110) from a proximal end to a distal end, and has at least one intersection with the braided component (110). In the catheter reinforcement layer (100), by means of introducing one or more axial components (120a, 120b, 120c, 120d, 120e, 120f, 120g, 120h, 120j, 120k, 1201, 120m, 120n) into a reinforcement layer of an existing catheter, the axial modulus of the catheter is increased so as to avoid the axial deformation of the catheter caused from the axial shear stress of the tubular body of the catheter when a device is delivered in the tubular body of the catheter.

## Description

### TECHNICAL FIELD

The present invention relates to the field of medical instruments and, in particular, to a catheter reinforcement layer and a catheter including the catheter reinforcement layer.

### BACKGROUND

With the support of Digital Subtraction Angiography (DSA) system, minimally invasive interventional surgery is to deliver implanted medical devices or therapeutic drugs to the lesion with minimal trauma through the intravascular catheter delivery system, so that the lesion is treated mechanically or chemically. As an important part of the delivery system, catheters have been widely used in various minimally invasive interventional treatments. At present, such interventional procedures usually form the entrance by puncturing small blood vessels (such as femoral artery and radial artery). After passing through the entrance, the catheter is delivered along the blood vessel to the target lesion with the assistance of the sheath and guide wire. The catheter then acts as an access for other devices such as stents, coils, other catheters, etc.

At present, in order to meet the requirement of smooth delivery of catheters to the lesion during clinical use, existing medical catheters are usually designed as segments with different stiffness. For example, the proximal end is more rigid and the distal end is more flexible, that is, the stiffness gradually decreases from the proximal end to the distal end. The design of the flexibility and stiffness of different segments needs to be determined according to the anatomy of the blood vessel. The catheter with a flexible distal end is easier to pass through the tortuous blood vessel and reduces the risk of damage to the blood vessel. However, due to the material characteristics of the catheter, the axial elongation of the flexible catheter is higher, and it is easy to have an axial deformation, for example, being stretched to lengthen, under the action of axial force. Therefore, during clinical operation, when other devices are delivered in the catheter, the tubular body of the catheter will be subjected to axial shear stress. If the axial elongation of the tubular body of the catheter is much greater, it is more likely to be axially stretched under the force of the device delivered therein. The tubular body of the catheter may be deformed after being stretched, and it is difficult to release or retrieve the delivered device, which has a serious adverse effect on clinical operations.

### SUMMARY OF THE INVENTION

An object of the present invention is to provide a catheter reinforcement layer and a catheter to solve the problem that the existing catheter is likely to be axially elongated when a device is delivered in the catheter due to the great elongation of the catheter.

In order to solve the above-mentioned technical problem, the present invention provides a catheter reinforcement layer, comprising a braided component and at least one axial component, wherein the braided component is a mesh tube formed by braiding wires in a crosswise manner, and the axial component extends along the braided component from a proximal end to a distal end, and has at least one intersection with the braided component.

Optionally, a material of the axial component is metal and/or polymer filament.

Optionally, the axial component has a filamentous structure shaped as any one or a combination of a straight line, wave line or spiral line.

Optionally, the axial component is arranged parallel to an axial direction of the braided component; or
the axial component is arranged at an angle of 0-45° with an axial direction of the braided component; or
at least a part of the axial component is arranged at an angle of 0-45° with an axial direction of the braided component, and other parts of the axial component are parallel to the axial direction of the braided component.

Optionally, the axial member has 1-45,000 said intersections with the braided component.

Optionally, the braided wires of the braided component are braided in the crosswise manner to form a mesh, and mesh intersections are formed at positions where the wires intersect, wherein,
all the at least one intersection of the axial component coincides with the mesh intersections; or
a part of the at least one intersection of the axial component coincides with the mesh intersections; or
none of the at least one intersection of the axial component coincides with the mesh intersections.

Optionally, the axial component, from the proximal end to the distal end, is shaped as a straight line or spiral line attached to an inner surface or an outer surface of the braided component; or
the axial component, from the proximal end to the distal end, is shaped as a wave line attached between an inner surface and an outer surface of the braided component in a staggered manner; or
the axial component, from the proximal end to the distal end, is arranged in the mesh of the braided component, and the axial component and the braided component are in a same plane.

Optionally, a plurality of said axial components is comprised; wherein
the plurality of axial components are arranged symmetrically or asymmetrically along a circumference of the braided component; and/or
the plurality of axial components are sequentially arranged at intervals along an axial direction of the braided component.

Optionally, a plurality of said axial components is comprised, and the plurality of axial components are arranged spirally along an axial direction of the braided component.

Optionally, axial distances between the plurality of axial components are the same or different, and/or
circumferential angular distances between the plurality of axial components are the same or different.

Optionally, the axial distances between the axial components located at the proximal end is smaller than the axial distances of the axial components located at the distal end; and/or
the circumferential angular distances between the axial components located at the proximal end is smaller than the axial distances of the axial components located at the distal end.

Optionally, a number of the axial components ranges from 1 to 16,000.

Optionally, an axial distance between two axial components adjacent in an axial direction of the braided component is in a range of 0.001-0.1 inches.

Optionally, a material of at least one of the at least one axial member is a radioautographic material.

Optionally, the axial component is a monofilament or a twisted filament composed of a plurality of monofilaments.

Optionally, a diameter of the monofilament is in a range of 0.0005-0.003 inches, and a number of the monofilaments contained in the twisted filament is in a range of 1-20.

Optionally, the axial component is connected to the braided component by means of gluing, polymerization, welding or heating, or is integrally formed with the braided component by means of braiding.

The present invention also provides a catheter, comprising an inner layer, a reinforcement layer and an outer layer that are sequentially arranged from inside to outside and are all in a shape of a tube, and wherein the reinforcement layer comprises the above catheter reinforcement layer.

Optionally, the catheter comprises a plurality of catheter segments connected in series, the axial component being arranged at a predetermined position; wherein
the predetermined position is between a circumferential surface of the braided component or a clearance of the braided wires where joint seams of adjacent catheter segments are located; or
the predetermined position is between a circumferential surface of the braided component or a clearance of the braided wires where a modulus value of a catheter segment is lower than a modulus value of an adjacent catheter segment at one or both sides; or
the predetermined position is at a part of the inner or outer layer of the catheter with a smaller thickness, or between a circumferential surface of the braided component or a clearance of the braided wires where a flexibility of a catheter segment is higher; or
the predetermined position is between a circumferential surface of the braided component or a clearance of the braided wires where a diameter of the catheter segment is smaller than a diameter of an adjacent catheter segment at one or both sides.

Optionally, the inner layer is a polymer material layer having a thickness of 0.0001-0.002 inches.

Optionally, the inner layer has a thickness of 0.0003-0.0006 inches.

Compared to the prior art, the present invention offers the following advantages:
1. The catheter reinforcement layer and catheter according to the present invention have a novel reinforcement layer, which increases the axial modulus of the catheter by introducing axial components into the reinforcement layer of the existing catheter. It avoids the axial deformation of the catheter caused from the axial shear stress of the tubular body of the catheter when a device is delivered in the tubular body of the catheter. In this way, the axial tensile capacity of the catheter is improved. Therefore, the elongation of the catheter is reduced, which avoids the risk of fatigue failure of the catheter caused from axial stretch by the compatible device.
2. One or more axial components is arranged on the mechanically weak point of the catheter, it is thus possible to prevent stress concentration on the catheter, thereby preventing the deformation of the tubular body of the catheter during the delivery of a device.
3. Introducing one or more axial components extending in the axial direction into the catheter reinforcement layer can improve the efficiency in transmission of the axial force of the catheter and optimize the transmission performance of the catheter.

### BRIEF DESCRIPTION OF THE DRAWINGS

Fig. 1 is a structural schematic diagram of a catheter reinforcement layer and a catheter using the catheter reinforcement layer according to an embodiment of the present invention;
Fig. 2 is a schematic plan view of the catheter reinforcement layer expanded along the axial direction according to an embodiment of the present invention;
Figs. 3a-3c are structural schematic diagrams of a catheter reinforcement layer according to another embodiment of the present invention;
Fig. 4 is a structural schematic diagram of a catheter reinforcement layer according to another embodiment of the present invention;
Fig. 5 is a structural schematic diagram of a catheter reinforcement layer according to another embodiment of the present invention;
Fig. 6 is a structural schematic diagram of a catheter reinforcement layer according to another embodiment of the present invention;
Fig. 7 is a structural schematic diagram of a catheter reinforcement layer according to another embodiment of the present invention;
Fig. 8 is another schematic plan view of the catheter reinforcement layer expanded along the axial direction according to an embodiment of the present invention.

List of reference numerals:
100, catheter reinforcement layer; 200, outer layer;
110, braided component; 120, axial component.

### DETAILED DESCRIPTION

The catheter reinforcement layer and the catheter including the catheter reinforcement layer of the present invention will be further described in detail below. The invention will now be described in more detail with reference to the accompanying drawings, in which preferred embodiments of the invention are shown. It should be understood that those skilled in the art may modify the invention described herein and still achieve the beneficial effects of the invention. Therefore, the following description should be understood as the broad knowledge of those skilled in the art, but not as a limitation of the present invention.

In the interest of clarity, not all features of a practical implementation are described. In the following description, well-known functions are not described in detail since they would obscure the invention in unnecessary detail. It should be appreciated that in the development of any practical embodiment, numerous implementation details must be worked out to achieve the developer's specific goals, such as changing from one embodiment to another in accordance with system-related or business-related constraints. Additionally, it should be recognized that such a development effort might be complex and time consuming, but would nevertheless be merely a routine undertaking for those skilled in the art.

As used herein and in the appended claims, the singular forms of "a", "an", and "the", include plural references unless the context clearly dictates otherwise. As used herein and in the appended claims, the term "or" refers to "and/or" unless the context clearly dictates otherwise. The term "plurality" is usually used to include two or more objects, unless the content clearly states otherwise. The term "several" is generally used to include an indefinite number of objects, unless the content clearly states otherwise. The term "proximal end" generally refers to the end close to the operator of the medical device, and "distal end" generally refers to the end of the device that first enters the human body, unless the content clearly indicates otherwise.

As mentioned in the background, at present, in order to meet the requirement of smooth delivery of the catheter to the lesion in clinical use, the existing catheters are usually designed as segments with different hardness. For example, the proximal end is harder and the distal end is softer, that is, the hardness gradually decreases from the proximal end to the distal end. The design of the softness and hardness of different segments needs to be determined according to the anatomy of the blood vessel. The catheter with a soft distal end is easier to pass through the tortuous blood vessel and reduces the risk of damage to the blood vessel. However, due to the material characteristics of the catheter, the axial elongation of the soft catheter is higher, and it is easy to have an axial deformation, for example, being stretched to lengthen, under the action of axial force. Therefore, during clinical operation, when other devices are delivered in the catheter, the tubular body of the catheter will be subjected to axial shear stress. If the axial elongation of the tubular body of the catheter is much greater, it is more likely to be axially stretched under the force from the device delivered therein. The tubular body of the catheter may be deformed after being stretched, and it is difficult to release or retrieve the delivered device, which has a serious adverse effect on clinical operations.

In view of the above problems, the researchers of the present invention found that the overall mechanical properties of the catheter are related to the modulus and stiffiness of the polymer material, and the strength of the metal wire of the reinforcement layer and the metal coverage. In the prior art, the catheter is mainly composed of an inner layer, a reinforcement layer and an outer layer, which form a triple-layer structure. For catheters with a reinforcement layer having a braided structure, the ability to resist fracture in the axial direction is relatively strong, and it is not easy to be broken when stretched. The axial elongation of the catheter depends on the polymer materials of the inner and outer layers. Therefore, when the inner and outer layers of the catheter are made of low-hardness polymer materials, such as thermoplastic elastomer (TPE), polyolefin, polyolefin elastomer or other materials, the catheter will have a decreased hardness due to the elasticity of the material. Additionally, an increased axial elongation of the catheter is achieved, so it is easy to be stretched and thinned under the axial force, which may easily cause the situation that the internal compatible device cannot be pushed, released or withdrawn during clinical use, affecting the surgical effect.

Therefore, the present invention provides a catheter reinforcement layer and a catheter including the catheter reinforcement layer to solve the problem that the existing catheter is likely to be axially elongated when a device is delivered in the catheter due to the great elongation of the catheter.

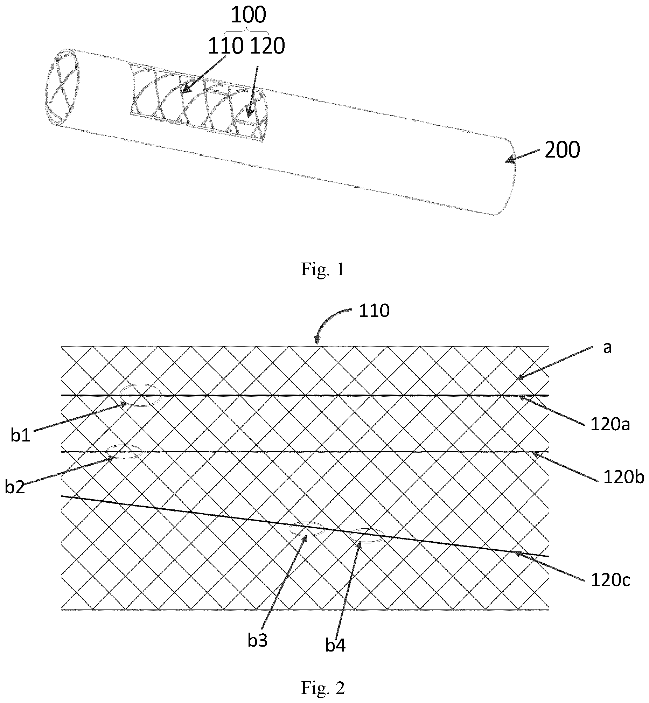

Referring to Fig. 1, which is a structural schematic diagram of a catheter reinforcement layer and a catheter using the catheter reinforcement layer according to an embodiment of the present invention. As shown in Fig. 1, the present invention provides a catheter, which can be used as a delivery catheter or for other medical purposes. The catheter has, for example, a hollow tube. In this embodiment, the catheter is, for example, a delivery catheter, which is used to provide a delivery path for an intravascular interventional device, and for release and withdrawal of the intravascular interventional device, and the like. The catheter includes, but is not limited to, a diameter of 6F-12F, so that the catheter can allow interventional instruments to be delivered into blood vessels in neurological interventional surgery, cardiac interventional surgery, aortic interventional surgery or peripheral vascular interventional surgery. In other embodiments, the diameter of the catheter can be modified according to actual needs.

As shown in Fig. 1, the present invention provides a catheter reinforcement layer and a catheter using the catheter reinforcement layer. The catheter includes an inner layer, a reinforcement layer and an outer layer which are sequentially arranged from the inside to the outside and are all in the shape of a tube. The catheter reinforcement layer includes a catheter reinforcement layer that includes a braided component and at least one axial component. The braided component is a mesh tube formed by braiding wires in a crosswise manner, and the mesh tube includes a mesh formed by braiding a plurality of wires in a crosswise manner, and mesh intersections are formed at the positions where the wires intersect. The axial component extends from the proximal end to the distal end along the braided component, and has at least one intersection with the braided component.

Specifically, refer to Fig. 2, which is a schematic plan view of the catheter reinforcement layer expanded along the axial direction according to an embodiment of the present invention. As shown in Fig. 2, the braided component 110 may include mesh cells formed by braiding a plurality of wires in a crosswise manner, and given that each of the intersections between the wires refers to the intersection a. All the intersections between the axial components and the braided component 110, such as the intersections b1 between the axial component 120a and the braided component 110, may coincide with the intersections a of the braided component 110. Optionally, all the intersections between the axial component and the braided component 110, such as the intersections b2 between the axial component 120b and the braided component 110, may not coincide with the intersections a of the braided component 110. Optionally, some of the intersections between the axial component and the braided component 110, such as the intersections b3 between the axial component 120c and the braided components 110, may coincide with the intersections a, and other ones of the intersections, such as the intersections b4 between the axial component 120c and the braided components 110, may not coincide with the intersections a of the braided components 110.

Optionally, in the embodiment of the present invention, there may be 1-45000 intersections between the axial component and the braided component. The specific number of intersections between each axial component and the braided component can be determined according to actual needs, which is not limited in the present invention.

Continuing to refer to Fig. 2, given that the end on the left side refers the proximal end, the axial components included in the braided component 110 extend from the proximal end to the distal end. The axial components, such as the axial component 120a and the axial component 120b, may be arranged parallel to the axial direction of the catheter. Optionally, axial components, such as the axial part 120c, may also be arranged at a certain angle, e.g., of 0-45° to the axial direction of the catheter. Moreover, when the braided component 110 includes a plurality of axial components, a part of the axial components may be arranged parallel to the axial direction of the catheter, while other parts may be arranged at a certain angle to the axial direction of the catheter, and the certain angle may include any angle within 0-45°, such as 3°, 5°, 10°, 15°, 30°, 45°. When a part or all of the axial components are arranged at a certain angle with the axial direction of the catheter, a same axial component may have same or different angles with the axial direction of the catheter in the length direction, and different axial components may have same or different angles with the axial direction of the catheter. The braided component includes a plurality of mesh cells connected in sequence, and the vertices of the mesh cells are the intersections between wires in the braided component. At least one of the axial components partially passes through at least one of the mesh cells of the braided component, and/or at least one of the axial components completely passes through one of the mesh cells or a plurality of continuous mesh cells of the braided component. The axial component may have a filamentous structure, and be shaped as a straight line, wavy line or spiral line or a combination thereof. Moreover, the braided component may be formed uniformly or non-uniformly along the axial direction of the catheter, which is not limited in the present invention.

The material of the inner layer of the catheter may be any one or any combination of polytetrafluoroethylene, polyurethane, polyamide, polyolefin, polyolefin elastomer and thermoplastic elastomer. The material of the outer layer of the catheter may be any one or any combination of polyurethane, polyolefin, polyolefin elastomer, thermoplastic elastomer and polyamide. In an exemplary embodiment of the present invention, the material of the inner layer and the material of the outer layer are preferably thermoplastic elastomers. The material of the braided component of the catheter reinforcement layer may be metal, for example, any one or any combination of stainless steel, nickel-titanium alloy, cobalt-chromium alloy and tungsten. Optionally, the material of the braided component may be polymer filaments, for example, made from any one or any combination of polyethylene, polyamide, liquid crystalline polymer. Optionally, the material of the braided component may be a combination of metal and polymer filaments. Moreover, the material of the axial component of the catheter reinforcement layer may be metal and/or polymer filaments. Specifically, the material of the axial component may be any one or any combination of metals such as stainless steel, nickel-titanium alloy, cobalt-chromium alloy, tungsten, silver and gold, or any one or any combination of polymer filaments such as polyethylene, polyethylene, liquid crystals. Optionally, the material of the axial component may be a combination of the metal and the polymer filaments. In the case of multiple axial components, the material of some of the axial components may be metal, and the material of some of the axial components may be polymer filaments. In an exemplary embodiment of the invention, the material of at least one of the axial components is a radioautographic material.

It should be noted that the catheter according to the present invention is soft because the materials of the inner and outer layers are same as those of the existing catheter, while one or more axial components are provided on the catheter reinforcement layer in the axial direction of the braided component, thereby increasing the axial modulus of the catheter, and avoiding the axial deformation of the catheter caused from the axial shear stress of the tubular body of the catheter when a device is delivered in the tubular body of the catheter. In this way, the axial tensile capacity of the catheter is improved. Therefore, the elongation of the catheter is reduced, which avoids the risk of fatigue failure of the catheter caused from axial stretch by the compatible device. Therefore, as long as the axial component is provided in any way in the axial direction of the braided component, the object of the invention of the present invention can be achieved. The axial direction may refer to a direction at a certain angle in a range of 0-45° with the axial direction of the catheter.

Various combinations of braided component and axial components in the catheter reinforcement layer included in the catheter according to the present invention will be described in detail below with reference to the accompanying drawings.

### Embodiment 1

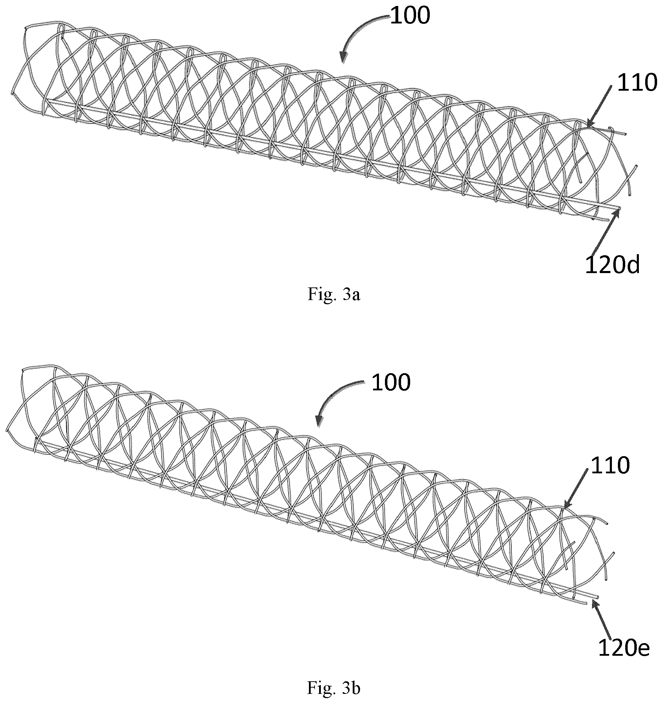

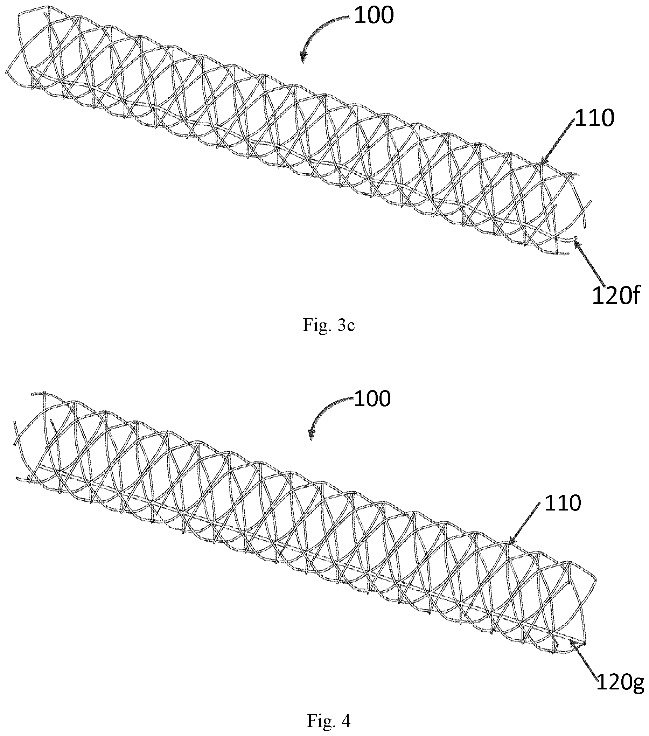

Referring to Figs. 3a-3c, which are structural schematic diagrams of a catheter reinforcement layer according to an embodiment of the present invention. As shown in Figs. 3a-3c as well as Fig. 1, the catheter reinforcement layer 100 includes a braided component 110 and an axial component 120 extending from the proximal end to the distal end along the axial direction of the braided component 110 and extending across the braided component 110. Specifically, as shown in Fig. 3a, the axial component 120d, from the proximal end to the distal end, may be shaped as a straight line or spiral line attached between the braided component 110 and the inner layer (not shown) of the catheter, that is, attached on the inner surface of the braided component 110. Alternatively, as shown in Fig. 3b, the axial component 120e, from the proximal end to the distal end, may be shaped as a straight line or spiral line attached between the braided component 110 and the outer layer (not shown) of the catheter, that is, attached to the outer surface of the braided component 110. Alternatively, as shown in Fig. 3c, the axial component 120f, from the proximal end to the distal end, may be shaped as a wave line attached to the inner surface and the outer surface of the braided component 110 in a staggered manner.

### Embodiment 2

Referring to Fig. 4, which is a structural schematic diagram of a catheter reinforcement layer according to an embodiment of the present invention. As shown in Fig. 4, the axial component 120g may be arranged in the mesh of the braided component 100 from the proximal end to the distal end, and the axial component 120g is in the same plane as the braided component 110 (i.e., each segment of the axial component is located in a corresponding mesh cell). In Embodiment 2, the axial component 120g may be connected to the braided component 110 by welding, bonding, etc., or the axial component 120d can be integrally formed with the braided component 110 by braiding. Compared with the catheter reinforcement layer 100 shown in Figs. 3a-3c, the catheter reinforcement layer 110 shown in Fig. 4 has an advantage in that the outer diameter of the catheter including the catheter reinforcement layer is not increased although the axial component 120 is included the catheter reinforcement layer.

It should be noted that, in addition to the case where the axial component 120 is provided in the manner described in Embodiment 1 and Embodiment 2 above (that is, the axial component 120 extends fully across the braided component 110 from the proximal end to the distal end) to form the catheter reinforcement layer 100 together with the braided component 110, the axial component 120 may alternatively extend partially across the braided component 110 to form the catheter reinforcement layer 100 together with the braided component 110. The invention is not specifically limited hereto.

In addition, one or more axial components 120 may be included. Specifically, when only one axial component 120 is included, the axial component 120 may be arranged on one side as described in Embodiment 1 and Embodiment 2. When a plurality of axial components 120 is included, the plurality of axial components 120 may be arranged symmetrically or asymmetrically along the circumference of the braided component 110, and/or the axial components 120 may be arranged at intervals along the axial direction of the braided component 110. It should be emphasized that the "interval" mentioned in this specification means that the proximal ends of two adjacent axial components are not at the same axial position, but arranged axially in a staggered manner. In some embodiments, two adjacent axial components may have no "overlap" in the axial direction. In other embodiments, two adjacent axial components may have partial "overlap" in the axial direction. The "overlap" here means that, at a same axial position, cross-sections of the above-mentioned two adjacent axial components are both located in the cross-section of the reinforcement layer.

The following provides examples where a plurality of axial components 120 are arranged symmetrically or asymmetrically along the circumference of the braided component 110 to introduce the positional relation between the axial components and the braided component 110. For details, see the following embodiments.

### Embodiment 3

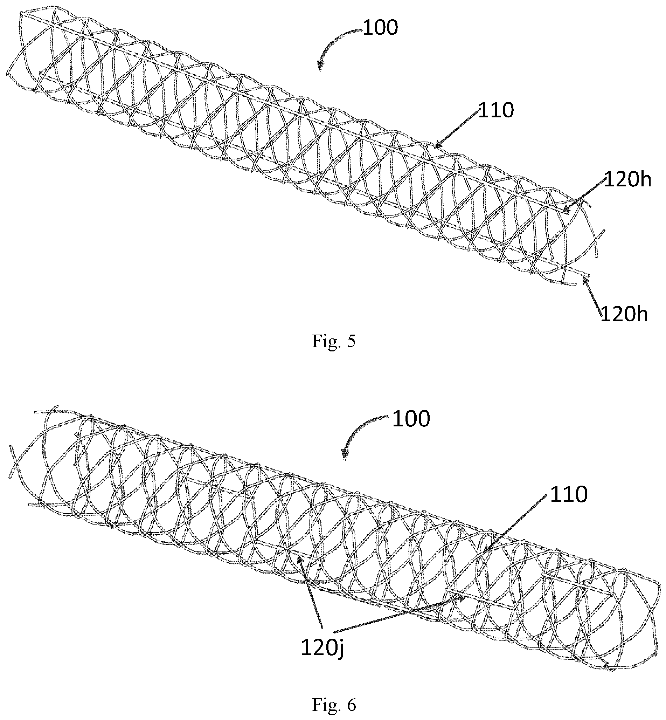

Fig. 5 is a structural schematic diagram of a catheter reinforcement layer according to an embodiment of the present invention. As shown in Fig. 5, preferably, the catheter reinforcement layer 100 includes a braided component 110 and a plurality of axial components 120h that extends across the braided component 110 from the proximal end to the distal end along the axial direction of the braided component 110 and are arranged symmetrically. Since the axial components 120 are disposed symmetrically on the surface of the braided component 100, the structural stability of the catheter including the catheter reinforcement layer can be ensured. Similar to Embodiment 1 and Embodiment 2, it avoids the axial deformation of the catheter caused from the axial shear stress of the tubular body of the catheter when a device is delivered in the tubular body of the catheter. Therefore, the elongation of the catheter is reduced, which avoids the risk of fatigue failure of the catheter caused from axial stretch by the compatible device.

Optionally, the number of axial components in the embodiment of the present invention may range from 2 to 10, for example, 2, 3, 5, 6, 8, or 10.

It can be understood that, when a plurality of axial components 120 are included, the plurality of axial components 120 can be arranged symmetrically or asymmetrically along the circumference of the braided component 110 at any position. However, in the prior art, the proximal end of the catheter is usually more rigid and the distal end is softer. In other words, the hardness gradually decreases from the proximal end to the distal end. In this case, a plurality of catheter segments with different hardness and/or thickness may be connected to form the catheter. The joints (joint seams or transitions) of the plurality of catheter segments and the thinner parts of the tubular body of the catheter tend to become weak points for stress. The weak points are prone to axial deformation or even torn off under the action of axial force during clinical operation. Therefore, in an embodiment of the present invention, a plurality of axial components 120 may be arranged symmetrically or asymmetrically along the circumference of the braided component 110 at predetermined positions, and/or the axial components 120 are sequentially arranged at intervals along the axial direction of the braided component 110 at predetermined positions. The predetermined positions may be at the junctions of the plurality of the catheter segments in the catheter reinforcement layer (i.e., joints or transitions of the inner layer or the outer layer), the positions where the inner layer or the outer layer has a higher flexibility, the positions where the thickness of the inner or outer layer is smaller, the positions where the modulus value of the catheter is lower, and the positions where the tubular body of the catheter is thinner.

### Embodiment 4

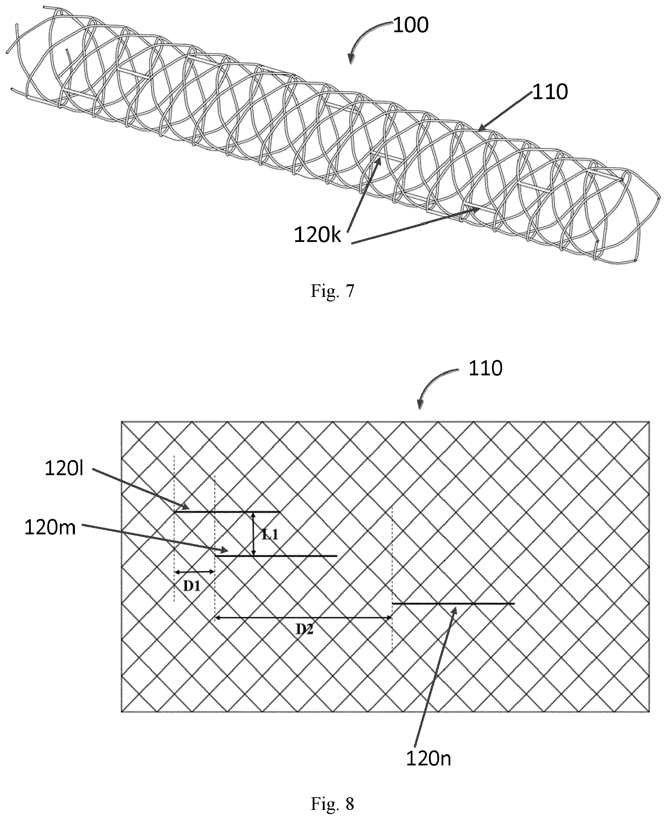

Referring to Figs. 6 and 7, which are structural schematic diagrams of a catheter reinforcement layer according to another embodiment of the present invention. As shown in Fig. 6 or Fig. 7, when a plurality of axial components 120 are included, the plurality of axial components 120k or axial components 120j may be arranged spirally along the circumferential direction of the braided component 110, and the plurality of axial components 120k or axial components 120j may be located between the circumferential surface of the braided component 110 or the clearance of the braided wires where the joint seams of adjacent catheter segments are located. Optionally, the plurality of axial components 120k or axial components 120j may be located between the circumferential surface of the braided component 110 or the clearance of the braided wires where the modulus value of the catheter segment is lower than that of the adjacent catheter segment at one or both sides, and between the circumferential surface of the braided component 110 or the clearance of the braided wires where the diameter of the catheter segment is lower than that of the adjacent catheter segment at one or both sides. Optionally, the plurality of axial components 120k or axial components 120j may be located at a part of the inner or outer layer of the catheter with a smaller thickness, or with a greater flexibility.

Specifically, each axial component 120j or axial component 120k may be arranged between the braided component 110 and the inner layer of the catheter, or between the braided component 110 and the outer layer 200 of the catheter. Optionally, a part of the axial components 120j or axial components 120k may be arranged between the braided component 110 and the inner layer of the catheter, and another part may be arranged between the braided component 110 and the outer layer of the catheter. Each axial component 120j or axial component 120k may be arranged between the mesh cells of the braided component 110. In addition, the axial length of each axial component 120 may be equal to the axial length of one of the mesh cells of the braided component 110, referring to the axial component 120k shown in Fig. 7. Optionally, the axial length of each axial component 120 may be equal to the axial length of a number (which is an integer or non-integer) of the mesh cells of the braided component 110, referring to the axial component 120j shown in Fig. 6. The present invention is not limited hereto. The plurality of axial components 120k or axial components 120j may be arranged spirally along the circumferential direction of the braided component 110, and the axial distance between plurality of axial components 120k or axial components 120j may be the same or different, and/or the circumferential angular distances of the plurality of axial components 120k or axial components 120j may be the same or different. Moreover, in the plurality of axial components 120k or axial components 120j, the axial distance of the axial components 120k or axial components 120j located at the proximal end of the braided component 110 may be smaller than that of the axial components 120k or axial components 120j located at the distal end of the braided component 110, and/or the circumferential angular distance of the axial components 120k or axial components 120j at the proximal end may be smaller than the axial distance of the axial components 120k or axial components 120j at the distal end.

In this embodiment, a plurality of axial components 120k or axial components 120j are arranged spirally along the circumferential direction of the braided component 110. In some other embodiments, the plurality of axial components 120k or axial components 120j may be located in the same circumferential direction, but spaced apart in the axial direction. In this embodiment, one or more axial components may be arranged on the mechanically weak point of the catheter, it is thus possible to prevent stress concentration on the catheter, thereby preventing the deformation of the tubular body of the catheter during the delivery of a device. Moreover, introducing one or more axial components extending in the axial direction into the catheter reinforcement layer can improve the efficiency in transmission of the axial force of the catheter and optimize the transmission performance of the catheter.

Optionally, the number of axial components in the embodiment of the present invention may be in the range of 1-16000, such as 1, 2, 4, 8, 20, 100, 800, 2000, 5000, 10000, 12000, 16000.

It should be noted that the proximal end of the braided component refers to the operator's side of the catheter including the catheter reinforcement layer. In other words, the proximal end of the catheter is also the proximal end of the braided component, the catheter reinforcement layer, and the axial component. Similarly, the distal end of the braided component refers to the side away from the operator of the catheter including the catheter reinforcement layer.

Optionally, refer to Fig. 8 in detail. Fig. 8 is another schematic plan view of the catheter reinforcement layer expanded along the axial direction according to an embodiment of the present invention. As shown in Fig. 8, given that the left side of the braided component 110 is the proximal end, the axial distance D1 between the two axial components 1201, 120m adjacent in the axial direction of the braided component 110, or the axial distance D2 between the adjacent axial components 120m, 120n may be in a range of 0.001-0.1 inches.

The axial distance refers to the distance between the proximal ends of two axial components in a direction parallel to the axial direction, and the circumferential angular distance refers to the radian corresponding to L1 (that is, the central angle formed from two points of two axial components on a same circumference).

Optionally, the axial component 120 may be a monofilament or a twisted filament composed of multiple monofilaments. The diameter of the monofilament is in a range of 0.0005-0.003 inches, and the number of monofilaments included in the twisted filament may be in a range of 1~20. Preferably, when the axial component 120 in the above embodiment is made from polyamide polymer filaments with a single filament size of 0.001 inches by means of polymerization, the effect on the soft catheter of the braided component 110 is excellent.

Exemplarily, the researchers of the present invention obtains the following table through experiments to show the influence of the axial component on various parameters of the catheter including the catheter reinforcement layer provided in the embodiment of the present invention. In this experiment, the specific arrangement of the axial component is shown in Fig. 3a, the axial component extends from the proximal end to the distal end along the axial direction of the braided component 110 and extends across the braided component 110. When the number of axial components is multiple (for example, N=2 or 3), the multiple axial components are arranged in parallel to each other.

**Table 1**

| Number of Axial Components | Elongation | Outer Diameter | Softness |
|---|---|---|---|
| N=1 | -38.0% | +0.1% | -3.1% |
| N=2 | -44.0% | +0.2% | -4.0% |
| N=3 | -56.0% | +0.4% | -7.0% |

It can be seen from the above Table 1 that, compared with the catheter without axial components, with the increase of the number of axial components, the axial elongation of the catheter gradually decreases, and the outer diameter and softness of the catheter remain basically unchanged. Therefore, adding axial components in the catheter can effectively reduce the axial elongation of the catheter with negligible influence on the outer diameter and softness of the catheter.

It can be understood that the axial components 120 shown in the figures can be connected to the braided component 100 by means of gluing, polymerization, welding or heating, or can be integrally formed with the braided component 110 by means of braiding in a specific way, such as by Triaxial Braiding.

In summary, the catheter reinforcement layer and the catheter according to the present invention have a novel reinforcement layer, which increases the axial modulus of the catheter by introducing axial components into the reinforcement layer of the existing catheter. It avoids the axial deformation of the catheter caused from the axial shear stress of the tubular body of the catheter when a device is delivered in the tubular body of the catheter. In this way, the axial tensile capacity of the catheter is improved. Therefore, the elongation of the catheter is reduced, which avoids the risk of fatigue failure of the catheter caused from axial stretch by the compatible device.

In addition, one or more axial components may be arranged on the mechanically weak point of the catheter, it is thus possible to prevent stress concentration on the catheter, thereby preventing the deformation of the tubular body of the catheter during the delivery of a device. Moreover, introducing one or more axial components extending in the axial direction into the catheter reinforcement layer can improve the efficiency in transmission of the axial force of the catheter and optimize the transmission performance of the catheter.

It should be noted that although the present invention has been disclosed above with preferred embodiments, the above embodiments are not intended to limit the present invention. For any person skilled in the art, without departing from the scope of the technical solution of the present invention, the technical content disclosed above can be used to make many possible changes and modifications to the technical solution of the present invention, or be modified into equivalent embodiments. Therefore, any simple modifications, equivalent changes and modifications made to the above embodiments according to the technical essence of the present invention, which do not deviate from the content of the technical solution of the present invention, still fall in the protection scope of the technical solution of the present invention.

It should also be understood that, unless otherwise specified or pointed out, the terms "first", "second", "third" and other descriptions in the specification are only used to distinguish each component, element, step, etc. in the specification, not used to express the logical relationship or sequence relationship between various components, elements, and steps.

In addition, it should be understood that the terminology described herein is used to describe particular embodiments only and is not intended to limit the scope of the invention. It must be noted that as used herein and in the appended claims, the singular forms "a" and "an" include plural referents unless the context clearly dictates otherwise. For example, a reference to "a step" or "a means" means a reference to one or more steps or means, and may include sub-steps as well as sub-means. All conjunctions used should be understood in their broadest sense. And, the word "or" should be understood as having the definition of logical "or" rather than logical "exclusive or", unless the context clearly expresses the contrary meaning. Additionally, implementation of the method and/or apparatus in embodiments of the present invention may include performing selected tasks manually, automatically, or in combination.

## Claims

1. A catheter reinforcement layer, comprising a braided component and at least one axial component, wherein the braided component is a mesh tube formed by braiding wires in a crosswise manner, and the axial component extends along the braided component from a proximal end to a distal end, and has at least one intersection with the braided component.

2. The catheter reinforcement layer according to claim 1, wherein a material of the axial component is metal and/or polymer filament.

3. The catheter reinforcement layer according to claim 1, wherein the axial component has a filamentous structure shaped as any one or a combination of a straight line, wave line or spiral line.

4. The catheter reinforcement layer according to claim 1, wherein the axial component is arranged parallel to an axial direction of the braided component; or
the axial component is arranged at an angle of 0-45° with an axial direction of the braided component; or
at least a part of the axial component is arranged at an angle of 0-45° with an axial direction of the braided component, and other parts of the axial component are parallel to the axial direction of the braided component.

5. The catheter reinforcement layer according to claim 1, wherein the axial member has 1-45,000 said intersections with the braided component.

6. The catheter reinforcement layer according to claim 1, wherein the braided wires of the braided component are braided in the crosswise manner to form a mesh, and mesh intersections are formed at positions where the wires intersect, wherein,
all the at least one intersection of the axial component coincides with the mesh intersections; or
a part of the at least one intersection of the axial component coincides with the mesh intersections; or
none of the at least one intersection of the axial component coincides with the mesh intersections.

7. The catheter reinforcement layer according to claim 1, wherein the axial component, from the proximal end to the distal end, is shaped as a straight line or spiral line attached to an inner surface or an outer surface of the braided component; or
the axial component, from the proximal end to the distal end, is shaped as a wave line attached between an inner surface and an outer surface of the braided component in a staggered manner; or
the axial component, from the proximal end to the distal end, is arranged in the mesh of the braided component, and the axial component and the braided component are in a same plane.

8. The catheter reinforcement layer according to claim 1, wherein a plurality of said axial components is comprised; wherein
the plurality of axial components are arranged symmetrically or asymmetrically along a circumference of the braided component; and/or
the plurality of axial components are sequentially arranged at intervals along an axial direction of the braided component.

9. The catheter reinforcement layer according to claim 1, wherein a plurality of said axial components is comprised, and the plurality of axial components are arranged spirally along an axial direction of the braided component.

10. The catheter reinforcement layer according to claim 9, wherein axial distances between the plurality of axial components are the same or different, and/or
circumferential angular distances between the plurality of axial components are the same or different.

11. The catheter reinforcement layer according to claim 10, wherein the axial distances between the axial components located at the proximal end is smaller than the axial distances of the axial components located at the distal end; and/or
the circumferential angular distances between the axial components located at the proximal end is smaller than the axial distances of the axial components located at the distal end.

12. The catheter reinforcement layer according to claim 1, wherein a number of the axial components ranges from 1 to 16,000.

13. The catheter reinforcement layer according to claim 1, wherein an axial distance between two axial components adjacent in an axial direction of the braided component is in a range of 0.001-0.1 inches.

14. The catheter reinforcement layer according to claim 1, wherein a material of at least one of the at least one axial member is a radioautographic material.

15. The catheter reinforcement layer according to any one of claims 1-14, wherein the axial component is a monofilament or a twisted filament composed of a plurality of monofilaments.

16. The catheter reinforcement layer according to claim 15, wherein a diameter of the monofilament is in a range of 0.0005-0.003 inches, and a number of the monofilaments contained in the twisted filament is in a range of 1-20.

17. The catheter reinforcement layer according to claim 1, wherein the axial component is connected to the braided component by means of gluing, polymerization, welding or heating, or is integrally formed with the braided component by means of braiding.

18. A catheter, comprising an inner layer, a reinforcement layer and an outer layer that are sequentially arranged from inside to outside and are all in a shape of a tube, and wherein the reinforcement layer comprises the catheter reinforcement layer according to any one of claims 1-17.

19. The catheter according to claim 18, comprising a plurality of catheter segments connected in series, the axial component being arranged at a predetermined position; wherein
the predetermined position is between a circumferential surface of the braided component or a clearance of the braided wires where joint seams of adjacent catheter segments are located; or
the predetermined position is between a circumferential surface of the braided component or a clearance of the braided wires where a modulus value of a catheter segment is lower than a modulus value of an adjacent catheter segment at one or both sides; or
the predetermined position is at a part of the inner or outer layer of the catheter with a smaller thickness, or between a circumferential surface of the braided component or a clearance of the braided wires where a flexibility of a catheter segment is higher; or
the predetermined position is between a circumferential surface of the braided component or a clearance of the braided wires where a diameter of the catheter segment is smaller than a diameter of an adjacent catheter segment at one or both sides.

20. The catheter according to claim 18, wherein the inner layer is a polymer material layer having a thickness of 0.0001-0.002 inches.

21. The catheter according to claim 18, wherein the inner layer has a thickness of 0.0003-0.0006 inches.
